Europäisches Patentamt

European Patent Office

Office européen des brevets

(11) Publication number: **0 091 945**
**B1**

(12) **EUROPEAN PATENT SPECIFICATION**

(45) Date of publication of patent specification: **25.05.88**

(51) Int. Cl.⁴: **A 61 B 1/26**

(21) Application number: **82903406.5**

(22) Date of filing: **15.10.82**

(86) International application number:
**PCT/US82/01419**

(87) International publication number:
**WO 83/01373 28.04.83 Gazette 83/10**

(54) **A LARYNGOSCOPE INCLUDING A SEPARATE DISPOSABLE BLADE AND ITS METHOD OF USE.**

(30) Priority: **16.10.81 US 311990**
**28.05.82 US 383030**

(43) Date of publication of application:
**26.10.83 Bulletin 83/43**

(45) Publication of the grant of the patent:
**25.05.88 Bulletin 88/21**

(84) Designated Contracting States:
**DE FR GB**

(56) References cited:
**EP-A-0 030 014**
**FR-A-2 361 855**
**US-A-2 289 226**
**US-A-2 648 329**
**US-A-3 281 637**
**US-A-3 426 749**
**US-A-3 598 113**
**US-A-3 739 769**
**US-A-3 766 909**
**US-A-3 826 248**
**US-A-3 916 881**
**US-A-3 949 740**
**US-A-4 273 112**

(73) Proprietor: **Upsher, Michael S.**
**2957 Adeline Drive**
**Burlingame California 94010 (US)**

(72) Inventor: **Upsher, Michael S.**
**2957 Adeline Drive**
**Burlingame California 94010 (US)**

(74) Representative: **Dr. E. Wiegand Dipl.-Ing. W.**
**Niemann Dr. M. Kohler Dipl.-Ing. J. Glaeser Dr.**
**H.-R. Kressin Patentanwälte**
**Flüggenstrasse 13**
**D-8000 München 19 (DE)**

# Description

The present invention relates generally to laryngoscopes and more particularly to a laryngoscope which utilizes a specifically designed disposable blade.

There are presently a number of generally similar types of laryngoscopes available in the prior art. The typical laryngoscope available includes an elongated handle, a separate blade for use on the handle, and an arrangement carried partially by the handle and partially by the blade for producing a beam of light in a predetermined direction relative to the blade. This latter arrangement utilizes a power supply contained within the handle for energizing a light source which produces the beam just recited. In most laryngoscopes, the light source is supported by and forms part of the blade, although in one type of laryngoscope presently being used the light source is carried by and forms part of the handle and cooperates with a light pipe on the blade. In either case, the handle and blade include cooperating means for disengagably connecting the two together so as to cause the power supply to energize the light source and cause the resultant beam to be pointed in the desired direction relative to the blade.

While laryngoscopes of the type described are generally satisfactory for their intended use, applicant has found that continued use of the same laryngoscope blade (1) requires sterilization which can be a costly procedure and (2) can result in cross-infection between patients.

In EP—A—30014 there is described a laryngoscope which should facilitate the elevating of the epiglottis of a patient and the inserting of an endotracheal tube into the throat and into the larynx of the patient. Furthermore, the known laryngoscope should make sure that the conductor wire between the battery and the light bulb of the laryngoscope does not become detached from the bulb, and the function of the bulb is not affected by moisture present when using the laryngoscope.

It is the object of the present invention to provide a disposable laryngoscope blade and associated handle which are designed to cooperate with one another so as to specifically discourage use of the blade more than once, but without interfering with its proper use the first time. This would avoid costly sterilization procedures and reduce the risk of cross-infections between patients.

This problem underlying the invention is solved by providing a laryngoscope comprising

(a) a handle including an elongated hand gripping portion and a blade connecting head portion;

(b) a disposable blade separate from said handle and including a handle connecting segment and an elongated tongue holding segment connected with and extending out from said handle connecting segment;

(c) means carried partially by said handle and partially by said blade for producing a beam of light in a predetermined direction relative to the tongue holding segment of said blade when said blade is connected with said handle, said beam producing means including a light source and power supply; and

(d) first and second cooperating means respectively forming parts of the head portion of said handle and the handle connecting segment of said blade for disengagably connecting said disposable blade to said handle, which laryngoscope, according to the invention, is characterized in that said second cooperating means include a readily severable, electrically conductive member forming part of the electrical circuit between said power supply and said light source and that said first and second cooperating means include means for severing a substantial part of said electrically conductive member as said blade is disengagably connected to said handle for the first time.

When the blade is connected to the handle for the first time this arrangement causes a component of the blade to be damaged sufficiently to discourage the use of the blade a second time without preventing it from being used in the proper way the first time.

In accordance with another feature of the present invention, the handle connecting and tongue holding segments of the blade are integrally molded as a single member which provides an elongated, open ended passageway configured to define an arc of a circle within a single plane. In this way, the passageway can be molded into the integral member using a core puller. A light guide is contained within the passageway and forms part of the arrangement for producing the previously recited beam of light. On the other hand, applicant has found that molding an integrally formed blade body with a passageway which defines an arc of a circle can be a relatively expensive task. Accordingly, as will be seen hereinafter, the present invention provides for a laryngoscope blade design designed in a way which minimizes this task.

The present invention provides a laryngoscope blade of the last-mentioned type and specifically one which includes a main body having a curved tongue holding segment and a light guide, at least a section of which corresponds generally in curvature to a section of the curved tongue holding segment.

Furthermore, the invention provides a method of making the last-mentioned laryngoscope blade in an uncomplicated and economical way and yet a way which includes integrally forming its main body to include a curved tongue holding segment and means for supporting its associated light guide in a similarly curved fashion without utilizing one or more curved holes in the blade body.

As will be described in more detail hereinafter, the laryngoscope blade disclosed herein in accordance with the invention is one which includes a handle connecting segment and an elongated, curved tongue holding segment connected with and extending out from the handle connecting segment. The blade also includes an elongated light guide, at least a section of which corresponds generally in curvature to a section of the curved tongue holding segment, and means for fixedly connecting the light guide to the handle connecting and tongue holding segments such that its curved section extends along and adjacent with the correspondingly curved section of the tongue holding segment. Moreover, the light guide is held in place without using a curved hole through either of the blade segments. Rather, in accordance with a preferred embodiment of the present invention, the two blade segments include respective straight passages for receiving opposite straight ends of the light guide in a way which causes an intermediate section of the latter to be supported in the necessary curved configuration. To this end, the light guide is sufficiently flexible to be bendable to different shapes but sufficiently rigid to hold its intended shape.

These and other features of the laryngoscope disclosed herein will be discussed in detail hereinafter in conjunction with the drawings, wherein:

Figure 1 is a side elevational view of a laryngoscope including a handle and disposable blade as well as other associated components designed in accordance with the present invention;

Figure 2 is a front longitudinal sectional view of the handle and certain associated components forming part of the laryngoscope of Figure 1;

Figure 3 is a partially broken away side elevational view of the handle and its associated components shown in Figure 2;

Figure 4 is an opposite side elevational view of a top portion of the handle illustrated in Figures 2 and 3;

Figure 5 is a side elevational view of the laryngoscope blade and certain associated components forming part of the laryngoscope of Figure 1;

Figure 6 is a top plan view of the blade and associated components illustrated in Figure 5;

Figure 7 is a bottom plan view of the blade and associated components shown in Figure 5;

Figure 8 is a front elevational view of the blade and associated components in Figure 5;

Figure 9 is a back elevational view of the blade and associated components shown in Figure 5;

Figure 10 is a partially broken away, side elevational view illustrating a top portion of the laryngoscope handle of Figure 1 and a back end segment of the blade showing how the latter is disengagably connected to the handle in accordance with the present invention;

Figure 11 is a view similar to Figure 10 but shows the laryngoscope blade in its disengagably connected position with respect to the handle;

Figure 11A is a view similar to Figure 11 but shows a prior art blade disengagably connected to the handle illustrated in Figures 2—4;

Figure 12 is a front elevational view of a top portion of a laryngoscope including a prior art handle and an arrangement for adapting the handle for use with the laryngoscope blade illustrated in Figures 5—9;

Figures 13—16 are various views of the adapter arrangement illustrated in Figure 12;

Figure 17 is a side elevational view of the laryngoscope blade designed in accordance with another embodiment of the present invention;

Figure 18 is a top plan view of the blade shown in Figure 17;

Figure 19 is a back end view of the laryngoscope blade illustrated in Figure 17;

Figure 20 is a front end elevational view of the laryngoscope blade illustrated in Figure 17;

Figure 21 is a plan view of the underside of the laryngoscope illustrated in Figure 17;

Figure 22 is an enlarged perspective view of a specific feature of the laryngoscope blade illustrated in Figure 17; and

Figure 23 is an enlarged perspective view of a modified version of the specific feature illustrated in Figure 22.

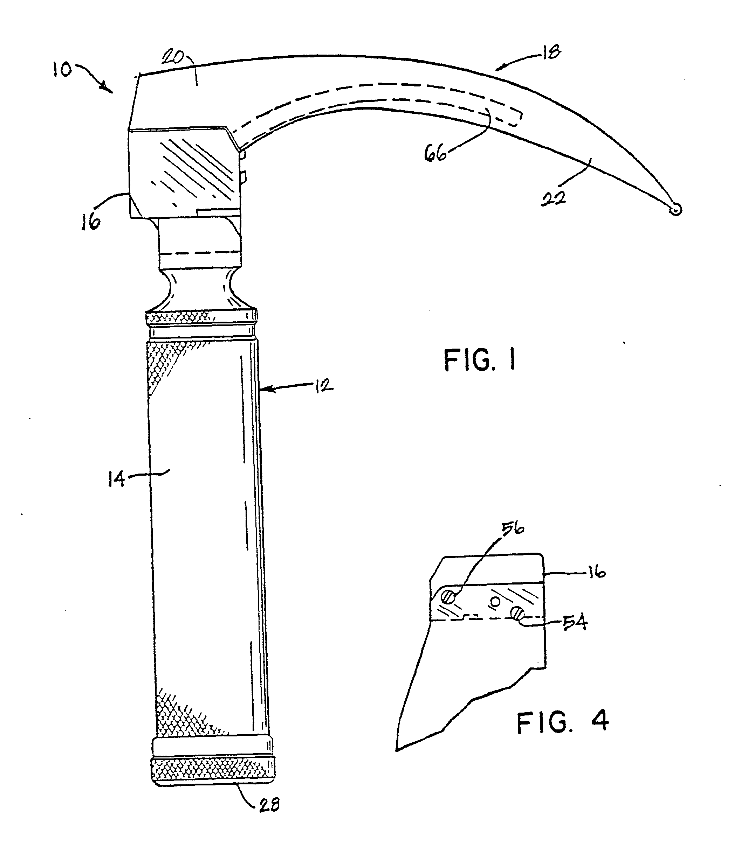

Turning now to the drawings, wherein like components are designated by like reference numerals throughout the various figures, attention is first directed to Figure 1 which illustrates a laryngoscope 10 designed in accordance with the present invention. This laryngoscope includes a handle 12 which may be divided into two functional portions, an elongated hand gripping portion 14 and a blade connecting head portion 16. The laryngoscope also includes a disposable blade 18 which is separate from the handle and which may be functionally divided into two segments, a handle connecting back end segment 20 and an elongated tongue holding segment 22 connected with and extending out from segment 20. In addition to handle 12 and disposable blade 18, laryngoscope 10 includes a number of associated components which together form a first arrangement for producing a beam of light in a predetermined direction relative to the blade when the latter is connected to the handle in a specific way and a second arrangement forming part of the blade and part of the handle for disengagably connecting the two together in a way which (1) produces the beam and (2) discourages use of the disposable blade more than once.

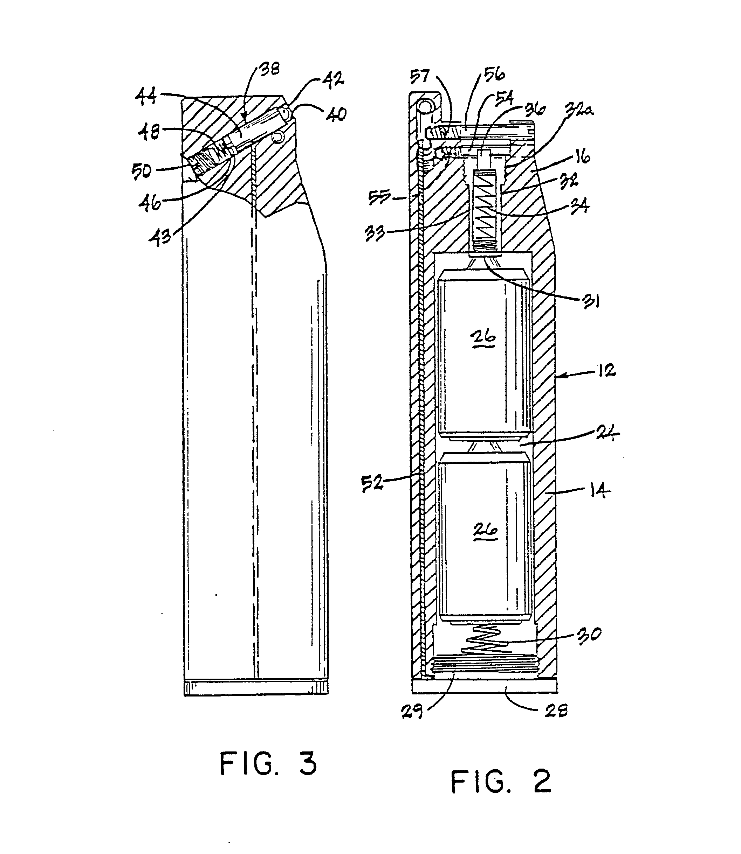

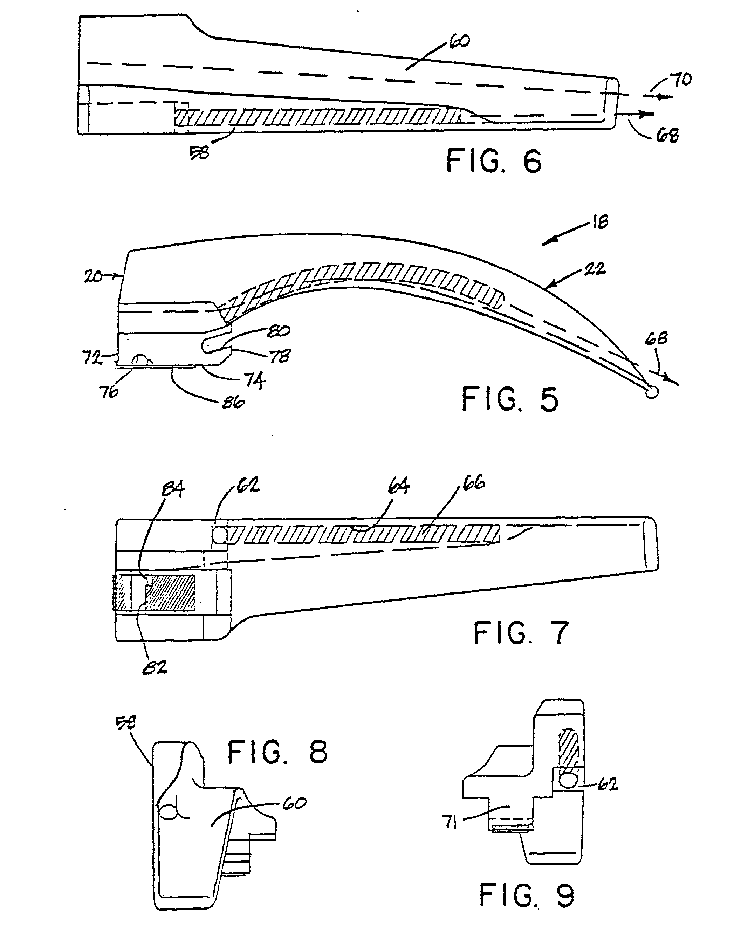

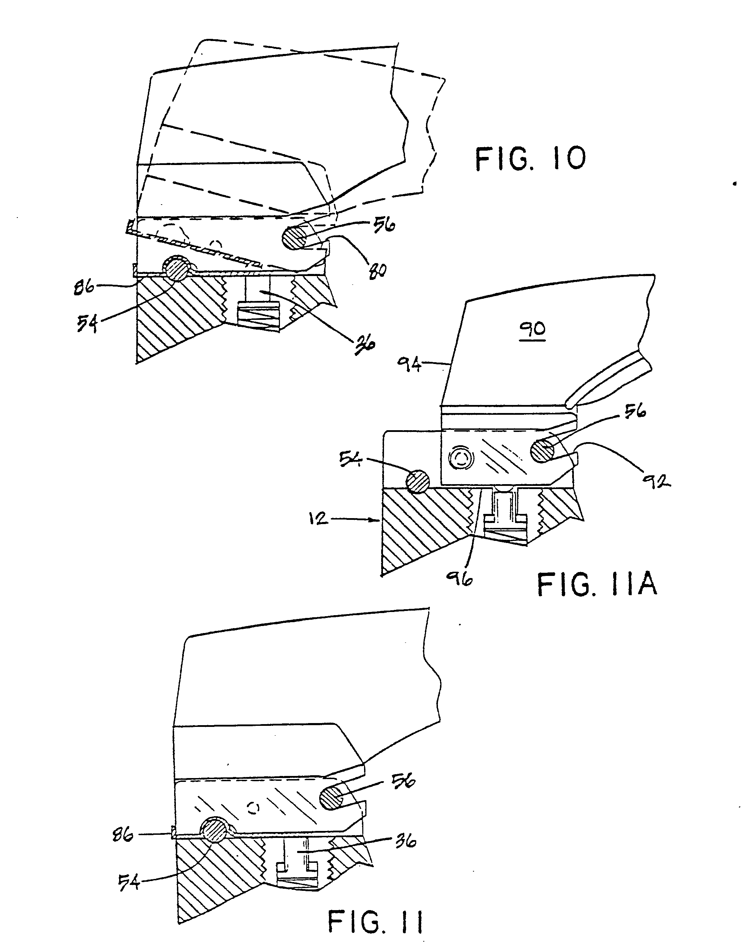

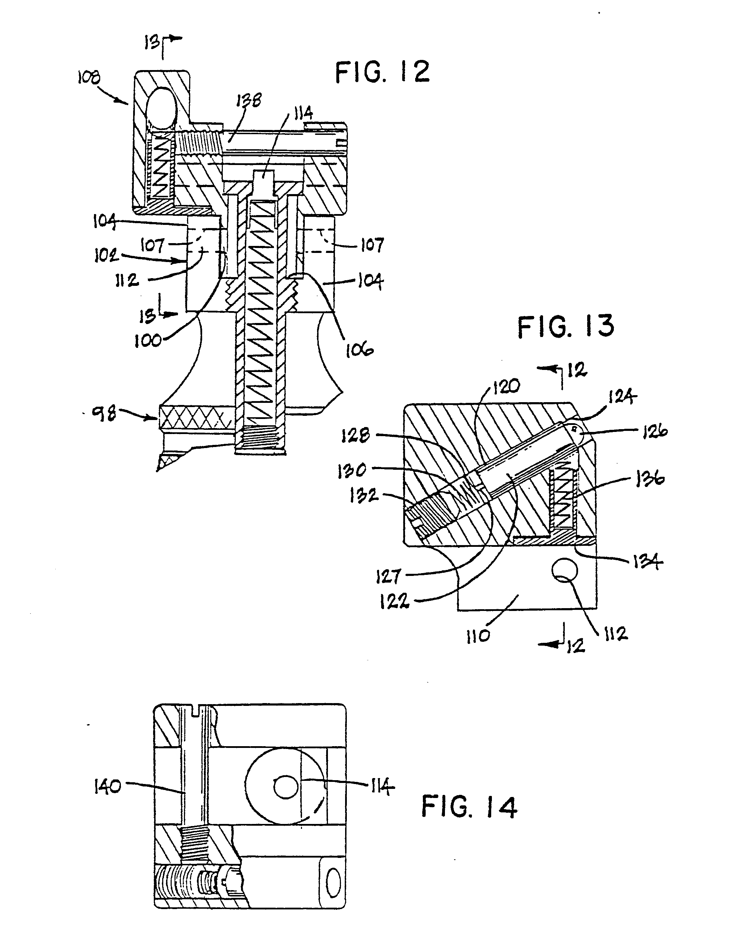

Referring to Figures 2—4, 10 and 11 in conjunction with Figure 1, attention is directed to handle 12 and a number of components forming part of the beam producing arrangement just recited. As illustrated specifically in Figures 2 and 3, the hand gripping portions 14 includes an interior chamber 24 which is opened at the bottom end of the handle for receiving and containing two series connected batteries 26. The bottom end of the container is sealed by means of an end cap 28 having an inner electrically conductive section 29 which is electrically connected to an adjacent battery 26 by means of an electrically conductive spring member 30. The spring member also serves to urge the two batteries in a vertically upward direction (as viewed in Figure 2) against an electrically conductive contact 31 at the bottom opened end of a stem or sleeve member 32. This latter member is contained within a cooperating internal opening 33 in end portion 16 of handle 12 and extends partially into chamber 24 as seen in Figure 2. A top end section 32a of sleeve 32 and a corresponding top end section of opening 33 are threaded in order to maintain the sleeve in place. In this regard, the top of the sleeve is slotted (not shown) to facilitate a screwdriver. The top end of contact 31 engages a second electrically conductive spring 34 which is also disposed within sleeve 32 and which extends up through and along the entire length of the sleeve. The top end of spring 34 engages and supports an electrically conductive actuating pin 36 for movement between a biased extended position illustrated in Figure 2 (and by dotted lines in Figures 10 and 11) and a retracted position illustrated by solid lines in Figures 10 and 11. When the pin 36 is in its extended position, it extends through a cooperating opening in the top end of sleeve 32. The batteries 26, section 29 of end cap 28 and its associated spring 30, the spring 34 and its associated acutator pin 36 together form part of the beam producing arrangement referred to above.

The beam producing arrangement also includes a suitable source of light, specifically the assembly generally indicated at 38 contained within a cooperating opening 40 in handle portion 16. Assembly 38 is composed of a conventional bulb 42 having a hot side contact 43 and a ground side contact (its body) contained within an electrically conductive grounding sleeve 44. The bulb body is maintained in electrical contact with the sleeve by means (not shown) within the sleeve. At the same time, contact 43 is engaged by a contact element 46 which is spring-loaded using the spring 48 and set screw 50, the latter also serving to close the back end of opening 40. The front end of opening 40 is left unobstructed so as to provide ready access to bulb 42. As illustrated in Figures 2 and 3, sleeve 44 is electrically connected to end cap section 29 by means of a ground wire 52 contained within and extending the length of handle 12 between the sleeve and end cap.

Referring specifically to Figures 10 and 11 in conjunction with Figure 2, the beam producing arrangement including the various components thus far described also includes a horizontally extending, fixed pin 54 which is disposed within a cooperating groove in the top of handle portion 16 and partially exposed at the top end of the handle. As best seen in Figure 2, the inwardmost end of pin 54 physically engages set screw 50 which means that pin 54 is in electrical contact with contact 46 which is in electrical contact with hot side contact 43 of bulb 42 and hence in electrical circuit with batteries 26 through the bulb filament and jacket and ground wire 52. Pin 54 is preferably threaded along its inward end section 58 adjacent set screw 50 and the opening in handle portion 16 containing this threaded end section is also threaded in a cooperating fashion.

The opposite end of pin 54 is preferably slotted as seen in Figure 4 so that a standard screwdriver can be used to place the pin in its proper position.

Pin 54 not only forms part of the beam producing arrangement but also serves as part of the previously recited arrangement for disengagably connecting blade 18 to handle 12. To this end, and for the reasons to be discussed, pin 54 is circular in cross section and is disposed a predetermined distance behind pin 36, as best seen in Figures 10 and 11. As also seen in these latter figures, a second horizontally extending cylindrical pin 56 is supported on and forms part of handle 16 immediately in front of and slightly above vertical pin 36. Pin 56 also forms part of the blade connecting arrangement just mentioned. At the same time, it serves as part of an alternate electrical circuit including batteries 26, pin 36 and bulb 42 but not including pin 54, as will be discussed hereinafter. For this reason, one end of pin 56, specifically its threaded end 57 which is contained within a cooperating threaded opening in the handle portion 16 is retained in physical engagement with bulb sleeve 44. The other end of pin 56 is slotted in order to place it in its operating position.

Referring now to Figures 5—9, attention is directed to disposable blade 18. As stated previously, this blade includes a handle connecting segment 20 and an elongated tongue holding segment 22 connected with and extending out from the handle connecting segment. In a preferred embodiment, these two segments are integrally formed as a single member by means of injection molding or the like and may be constructed of any material compatible with its method of manufacture, preferably hard plastic, for example, polycarbonate. As best illustrated in Figures 6 and 8, tongue holding segment 22 may be divided into two lengthwise sections, a tongue blocking and light guide holding section 58 forming a lengthwise side of segment 22 and a viewing and anesthesia tube guiding section 60 forming the other lengthwise side of the segment. Section 58 terminates rearwardly at a planar shoulder 62 (see Figures 7 and 9) which forms part of handle connecting segment 20. For reasons to become apparent hereinafter, shoulder 62 is disposed at an angle of about 30° with the vertical, that is, perpendicular to the axis of passageway 40 in blade connecting head portion 16 of handle 12 which means that shoulder 62 is perpendicular to the beam axis of light source assembly 38.

Section 58 of blade segment 22 includes an open ended, internal passageway 64 extending from the rearward end of the section at shoulder 62 to its front end which is best illustrated in Figure 8. This passageway is configured to define an arc of a circle (see Figure 5) which lies within a single plane (see Figures 6 and 7). This passageway serves to contain a light guide 66, for example, an optical fiber, a bundle of optical fibers, or the like, extending the entire length of the passageway. In an actual embodiment, a single rod of acrylic was used successfully. As will be seen hereinafter, light guide 66 cooperates with the light source 42 when blade 18 is connected with handle 12 for directing the beam produced by the light source in a predetermined direction relative to the blade, specifically, in the direction of arrow 68 illustrated in Figures 5 and 6. This light is used to illuminate a patient's throat during the initial anesthesiology workup while section 58 of the blade holds the tongue to one side. As a result, the anesthesiologist is provided with a clear view into the laryngeal area of the patient along section 60 and he may use this latter section as a guide to insert the anesthetic tube or tubes (not shown) into position.

The passageway 64 was described above as defining an arc of a circle. While blade 18 is not limited to this particular configuration, it is preferred for ease of manufacture when the blade is of the Macintosh type, e.g., curved. More specifically, by making the passageway truly arcuate and particularly less than that of a half circle, passageway 64 can be molded into the blade segments 20 and 22 as these segments are molded into a single member using a suitable shaped core puller. This cannot be done if the passageway is not either straight or truly arcuate since the core puller could not be removed once a curved but not arcuate passageway is formed. Because of the desired arcuate configuration of passageway 64 and therefore light guide 66, the latter cannot be bent out of its arcuate configuration to better coincide with section 60 during observation of the patient's larynx. Therefore, as best illustrated in Figure 6, section 60 is designed to extend at an acute angle with section 58 so that the observation angle along section 60, as indicated by arrow 70, is at an acute angle with light beam 68. In this way, the light beam more efficiently illuminates the area being observed.

Having described segment 22 of blade 18 and its associated light guide 66, attention is now directed to segment 20. As illustrated in Figures 5 through 9, this segment includes a rearward projection 71 having a vertically extending, flat back end 72 which is positioned directly behind section 60 of blade segment 22. Directly below section 60 and joining back end 72 is a horizontally extending, flat bottom 74 which contains a downwardly opening slot 76 to be discussed below. Opposite back end 72 and extending up from bottom 74 is a front end 78 of blade segment 20. Front end 78 includes an inwardly extending and slightly downwardly angled slot 80 (see Figure 5) extending across its entire width (see Figure 7). The slot 76 also extends the entire width of projection 71 (again, see Figure 7) and for reasons to be discussed hereinafter, the cross sectional configuration of this latter slot defines an arc of a circle which is slightly greater than a half circle. The forward edge of this slot is designed to provide a relative sharp, knife edge 82 along a portion of its length from one end thereof. The rest of the same edge is recessed as indicated at 84 therefore eliminating a cutting edge at that point. The entire slot 76 and a portion of bottom 74 are covered by an electrically conductive foil 86 bonded to bottom 78 or other electrically conductive means. For reasons to be discussed, the foil or other such means is readily severable in the way to be described.

Having now described laryngoscope blade 18 in its entirety from a structural standpoint. attention is directed to the way in which this blade is connected and interacts with handle 12, as best illustrated in Figures 10 and 11. As seen there, blade segment 20 is initially disposed over the top end of handle 12 at an angle with the latter. Pin 56 forming part of the blade connecting head portion 16 is located in slot 80 and is used as a means for pivoting the blade between the angled position illustrated in Figure 10 and the flat operating position illustrated in Figure 11. As blade segment 20 moves from its Figure 10 position to its Figure 11 position, a front end of foil 86 engages against and remains in contact with pin 36 as the latter is pushed down into its retracted position. At the same time, slot 76 is forced into snap fitting engagement around pin 54. In this latter regard, as stated previously, the diameter of slot 76 is approximately equal to the diameter of pin 54 but the cross section of the slot is greater than a half circle. Moreover, projection 71 which is preferably plastic is sufficiently resilient to provide a limited degree of give to slot 76 so that the latter can snap around and interlock with pin 54 upon application of sufficient force. At the same time, cutting edge 82 severs a corresponding length of the foil (or other such means) across the width of the latter. However, because of recess 84, foil 86 is not entirely severed. Thus, with blade 18 mounted on handle 12 in the manner illustrated in Figure 11, foil 86 serves as an electrical connection between pins 36 and 54. This, in turn, closes the electrical circuit between batteries 26 and light bulb 42 causing the latter to be energized for producing a beam of light. At the same time, the foil is sufficiently damaged as a result of being partially severed so as to discourage anyone from using the disposable blade a second time. While a foil is preferred to accomplish this, it is to be understood that other means serving the same function could be used.

From the foregoing, it should be apparent that the pins 36, 54 and 56 form a part of handle portion 16 and the slots 76 and 80 and foil 86 forming part of blade segment 20 cooperate with one another for disengagably connecting the disposable blade to the handle in a specific way so as to energize light bulb 42. At the same time, when the blade is used for the first time, a component thereof is damaged, specifically foil 86, sufficient to discourage anyone from using the blade a second time, but without preventing it from being used in the proper way the first time. In addition, placement of the laryngoscope blade on its handle in the appropriate way automatically places planar shoulder 62 and the back end of light guide 66 in confronting relationship with and across the front of opening 40 and light bulb 42. In this way, light guide 66 is able to capture and collect substantially all of the light beam passing out of the opening 40 from the light bulb for directing this beam in the direction of arrow 68. This is accomplished without having to provide a 90° bend in the light guide and yet the light is ultimately directed in the most beneficial direction relative to blade segment 22. Moreover, this has been found most easily accomplished by orienting opening 40 and therefore the axis of the light beam leaving this opening at an angle of 30° with a plane normal to the axis of handle portion 14, that is, with the horizontal when the handle portion is held in a vertical direction.

Having described laryngoscope 10, it should be apparent that the blade 18 can be made relatively economically by molding the segments 20 and 22 as a single integral member with the passageway 64 being formed of the same time. In this way, in order to complete the blade, all that is necassary is that the light guide be inserted into the passageway and that the foil 86 be adhered to bottom 74 of projection 71. Moreover, all of these components are relatively inexpensive and therefore the overall blade is disposable after a single use. To this end, by partially severing the foil when the blade is first used, its user is encouraged to dispose of it thereafter.

Because of the way in which blade 18 is designed, handle 14 is different than the typical prior art handle, at least to the extent that handle 14 includes pin 54 in circuit with batteries 26 and bulb 42. In the typical prior art handle, no such pin exists. Rather, the prior art handle utilizes a pin corresponding to pin 56 (and the rest of the blade body) in conjunction with a pin corresponding with pin 36 to close the electrical circuit between its batteries and associated light source and the latter is typically carried by the blade rather than the handle. In this latter case, the light source is connected in circuit with an insulated button on the blade which makes contact with pin 36. At the same time, the rest of the blade including pin 56 makes contact with the handle body and serves as a ground. Figure 11A illustrates the back end of a conventional metal laryngoscope blade generally indicated at 90. The handle connecting back end segment of this blade includes a front slot 92 corresponding to previously described slot 80, a back end 94 and a bottom 96. This handle connecting segment can be mounted to the top end handle 12 by placing pin 56 in slot 92 and pivoting the blade about the axis of this latter pin until the bottom 96 rests against pin 36 and causes this pin to move to its retracted position. This, in turn, incorporates the batteries 26 into and closes an electrical circuit including a light source associated with and carried by the blade 90 for energizing the light source. However, it should be noted that the pin 54 is positioned relative to pin 36 such that the back end 94 of blade 90 does not engage this rearward pin. Therefore, when prior art blade 90 is disengagably connected to handle 12, bulb 42 carried by the handle is not energized and therefore does not unnecessarily drain the batteries or needlessly withdraw power from the other, intended light source.

Having described the way in which handle 12 forming part of a laryngoscope 10 cooperates with a prior art blade, attention is now directed to the way in which the prior art handle can be readily converted to one which will operate with blade 18 in the same manner as handle 12. The top end of this prior art handle is generally illustrated in Figure 12 at 98. While not shown, the bottom end of the handle may be identical to handle portion 14 and includes similar batteries 26. The top end of the handle includes a pin 100 which corresponds to pin 56 of handle 12 that is, it is the pin which is disposed within the slot corresponding to slot 92 of prior art blade 94. While not shown, this conventional handle also includes a vertically extending pin and associated component corresponding to pin 36 and components 32 and 34 (see Figure 2). However, in order to adapt handle 98 to include blade 18, this latter pin and its associated components are removed and entirely eliminated and the pin 100 is temporarily disassembled from the blade connecting head portion 102 forming the top end of the handle. This leaves a pair of spaced apart flanges 104 having aligned through holes 107 extending vertically upward at the top end of the handle across which pin 100 is supported. With the pin disassembled therefrom, a threaded opening 106A into the base 106 of portion 102 is made accessible. This opening extends all the way down to the chamber in the handle containing batteries corresponding to batteries 26.

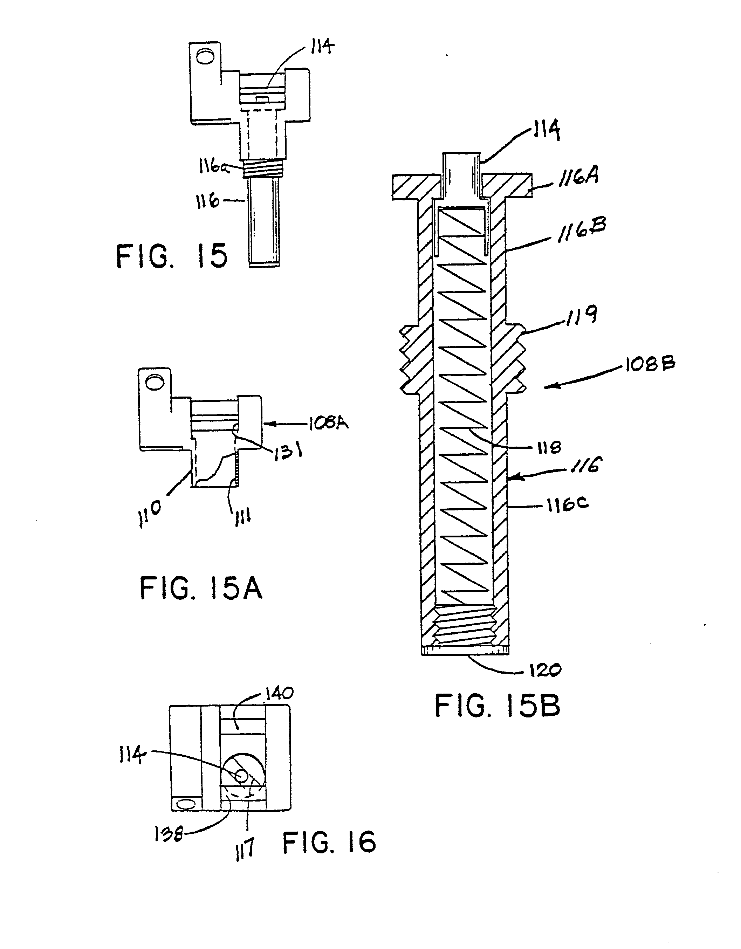

With portion 102 of handle 98 in the condition just recited, an adapter generally indicated at 108 is mounted on and connected with the handle portion. This adapter may be separated into two components, a main body 108A shown alone in Figure 15A and a shaft arrangement 108B shown alone in Figure 15B. Main body 108A includes a lowermost base section 110 (see Figures 13 and 15A) which includes a vertically extending unthreaded through hole 111, which fits snugly between flanges 104 (see Figure 12) and which inclues a horizontal through hole 112 in alignment with the through holes 107 of flanges 104. In this way, the pin 100 can be reassembled into its cooperating openings 107 and, at the same time, through opening 112 for holding the adapter in place. Also, the hole 111 is automatically placed in vertical alignment with and above treaded hole 106A in the handle.

As best illustrated in Figures 14, 15B and 16, shaft arrangement 108B includes its own vertically extending pin 114 corresponding in function to pin 36 disposed within a sleeve 116 corresponding to sleeve 32. As seen in Figure 15B, the sleeve 116 includes an enlarged head 116A which is slotted at 117 (see Figure 16), an intermediate section 116B which is threaded at 119 and a narrower bottom portion 116C. A spring 118 is also located within sleeve 16 between pin 114 and a lowermost contact 120 which is thread

connected into and through a cooperating threaded opening 122 in the bottom of sleeve 116. The lowermost unthreaded end of sleeve 116 is provided for maintaining pin 114 in electrical contact with the batteries in handle 98 when the adapter is in place on top of the handle. In this regard, the bottom portion 116C and the threaded section 119 of portion 116B of the shaft 116 are placed through hole 111 in main body 108A. The threaded section 119 cooperates with threaded hole 106A in base 106 of handle 98 so that contact 120 engages the batteries in the handle. In this latter regard, the slot 117 in enlarged head 116A is used to thread connect section 119 with threaded opening 106A until the head moves into a cooperating recess 131 in main body 108A (see Figure 15A). Thus, the bottom threaded section 119 of sleeve 116 in Figure 15 is thread connected into the opening in base 106 for connecting the adapter to the handle. Pin 114, the spring 118 and contact 120 together function in the same manner as the pin 36 and its associated components 31, 32 and 34.

Referring specifically to Figures 12—14, the adapter's main body 108A is shown including a light source assembly 120 which may be identical to the previously described assembly 44. Thus, assembly 120 includes a metal grounding sleeve 122, contained within an open ended passageway 124 in the adapter and housing the light bulb 126 which remains in place by means of a shoulder at its forwardmost end. A hot side contact 129 on the bulb, a contact 128, an associated spring 130 and adjacent set screw 132 are provided and correspond to the components 43, 46, 48 and 50 in Figure 3. In addition, the adaptor includes a grounding rivet 134 which contains and is in electrical contact with a spring 136. The spring also engages against sleeve 122, thus placing the ground side of light bulb in electrical contact with the sleeve.

In addition to the various components thus far described, adapter 108 includes a front pin 138 disposed in front of and slightly above pin 114 (see Figure 14) and a back pin 140 positioned directly behind pin 114. The pin 138 corresponds in function and structure to previously described pin 56 and the pin 140 corresponds in function and structure to pin 54. In other words, the three pins 114, 138 and 140 respectively serve the same purposes as the pins 36, 56 and 54 in supporting laryngoscope blade 18. In order to provide the appropriate electrical connections between these various components, the pin 138 is designed to engage the rivet 134 as illustrated in Figure 12. This places pin 138 in electrical contact with sleeve 122 and therefore the ground side of bulb 126. The pin 140 physically engages the set screw 132 (see Figure 14), thereby placing pin 140 in electrical contact with the hot side contact 127 of bulb 126. By providing the combination rivet 134 and spring 136, it is not necessary to directly engage against sleeve 122 with pin 138, thereby minimizing damage to the latter. This is also true for pin 140 which engages the set screw 132 rather than sleeve 122.

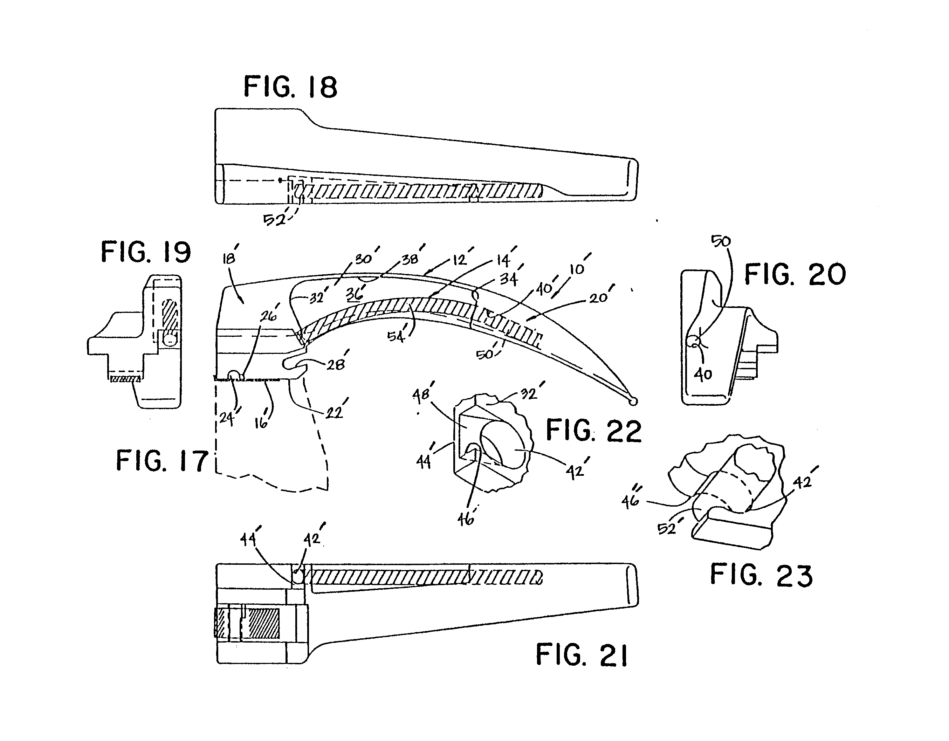

Turning now to Figures 17—23, wherein like components are designated by like reference numerals throughout the various figures, attention is first directed to Figure 17 which illustrates a larngoscope blade 10' designed in accordance with the present invention. The blade is shown in Figure 17 including a main body 12', an elongated light guide 14' and a strip of electrically conductive material 16'. Each of these components functions in the manner to be described hereinafter. For purposes of description, the laryngoscope body 12' may be separated into two segments, a handle connecting back segment 18' and an elongated, curved tongue holding forward segment 20' connected with and extending in front of segment 18'.

As seen best in Figures 17 and 21, electrically conductive strip 16' which is, for example, a metal foil extends across and is connected with the bottommost, downwardly facing surface 22' forming part of handle connecting segment 18'. The strip covers a longitudinal portion of a slot 24' and indent 26' extending into segment 18 from surface 22'. Segment 18' is also shown including a second slot 28' located above surface 22' and facing in the forward direction under tongue holding segment at a slightly upward incline. Strip material 16' in cooperation with slots 24' and 28' and indent 26' serve to disengagably connect the handle connecting segment 18' to a cooperating blade connecting head portion forming part of a laryngoscope handle (indicated by dotted lines in Figure 17) in the manner described above (see Figures 1—16). It should suffice to say that these components and the particular way in which the blade is connected to the handle and the handle itself do not form part of the present invention. In this regard, it is to be understood that the laryngoscope blade designed in accordance with the present invention could be readily designed to include a different means of connection with a cooperating handle and may or may not include strip material 16 and the combination of slot 24' and indent 26' which together are intended to discourage a person from using the blade more than once.

The laryngoscope body 12' is an integrally formed member consisting of handle connecting segment 18' and tongue holding segment 20'. In a preferred embodiment, the body is suitably molded from plastic, although it could be constructed of stainless steel or other metals. As seen best in Figure 17, the tongue holding segment defines an arcuate path from its back end to its front end so as to be readily insertable into the throat of a patient. For reasons to be discussed below, this arcuately shaped segment includes an elongated pocket 30 which extends into but not entirely through one side of segment 20'. This pocket is defined by a forwardly facing inclined back wall 32 forming part of segment 18', a rearwardly facing inclined front wall 34', an innermost side wall 36 and a downwardly facing top wall 38', the latter three walls forming part of segment 20'.

As illustrated in the various figures, an open-ended, straight hole 40' extends between front wall 34' and the front face of tongue holding segment 20' (see Figure 20). This hole is circumferentially closed along its entire length and extends at an acute angle upwardly from its front end to its back end. A second open-ended, straight hole 42' is provided and extends between back wall 32' and a rearward face 44' forming part of segment 18'. This latter opening is best illustrated in Figure 22 and is shown extending at an acute angle upwardly from its back end to its front end. In addition, a slot 46' defined by ramped surface 48' extends into opening 42' from one side thereof.

The holes 40' and 42' serve to maintain the previously recited light guide 14' in a fixed position on body 12' of the laryngoscope body. More specifically, hole 40' serves to receive a straight front section 50 of the light guide while hole 42' serves to receive a straight back end section 52'. Since the light guide is flexible and because of the relative positions of holes 40' and 42' relative to one another and to the rest of the laryngoscope body, an intermediate section 54' of the light guide is disposed within pocket 30' and maintained in a curvilinear configuration corresponding to and extending along and adjacent to a section of the curved tongue holding segment 20', as best illustrated in Figure 17. Because the openings 40' and 42' are straight, the entire laryngoscope body can be molded as an integral member much more economically than if arcuate holes are used, as in the case of the laryngoscope blade illustrated in Figures 6—9.

Having described laryngoscope blade 10', attention is now directed to one way in which light guide 14' is assembled with body 12'. The first step in this preferred process is to insert front end section 50' of the light guide into hole 40' until the front end of the light guide is approximately flush with the front end of the hole. Thereafter, intermediate section 54' is bent into the curved configuration illustrated so as to place the rearwardmost end of the light guide against ram 48' of slot 46'. The back end of the light guide is then forced inward along the ramp until back section 52' of the light guide snaps into hole 42'. The light guide will remain in this position. When the blade is mounted to its associated laryngoscope handle in the normal operating manner, the back end of light guide 14' is automatically placed in optical alignment with the light source carried by the handle which is automatically energized by a power supply in the handle as described in the Figure 1—16 embodiment above. In this way, the light guide serves to redirect light from its optically aligned source toward its front end. This light passes out the front end of the light guide and is used to illuminate an area in advance of the blade as the latter is inserted into a patient's throat.

In accordance with another, preferred way of assembling light guide 14', the latter is initially molded or otherwise formed into its ultimate shape shown in Figure 17. In this way, the straight front end section 50' can be inserted entirely into hole 40' until reaching the already curved section 54'. The back end of the light guide can then be assembled into place.

Referring to Figure 23, the hole 42' is shown including a modified slot 46''. This latter slot extends the entire length of hole 42 and therefore does not include a ramped surface 48. Moreover, the slot 46'' is slightly smaller vertically than the diameter of hole 42'. In this way, the back end section 52' of light guide 54' can be snapped into hole 42' through slot 46'' without being forced to ride on a ramped surface such as the one illustrated in Figure 22. Of course, in order to force end section 52' through slot 46'', either the material making up blade segment 18', at least around the slot, must provide sufficient resiliency to allow the slot to be expanded outward or section 52' of the light guide must be sufficiently resilient or compressible to adequately deform or compress sufficient to pass through the slot. In either case, it is assumed that the cross sectional configuration of end section 52' is larger than the vertical dimension of the slot which is preferable so that the end section cannot inadvartently slip out of opening 42' once in place therein.

While laryngoscope body 12' has been described including a straight, circumferentially closed opening 40' and a slotted opening 42', it is to be understood that two straight openings entirely closed circumferentially could be provided or two slotted openings could be used. Moreover, the light guides does not have to be flexible, although this is preferred for ease of assembly. It is only necessary that the holes, whether they are slotted or not, by straight so as to make the manufacture of the blade by means of molding relatively easy.

**Claims**

1. A laryngoscope (10, 10') comprising:

(a) a handle (12) including an elongated hand gripping portion (14) and a blade connecting head portion (16);

(b) a disposable blade (18, 12') separate from said handle (12) and including a handle connecting segment (20) and an elongated tongue holding segment (22, 20') connected with and extending out from said handle connecting segment (20, 18');

(c) means (26; 42, 126) carried partially by said handle (12) and partially by said blade (18, 12') for producing a beam of light in a predetermined direction relative to the tongue holding segment (22, 20') of said blade (18, 12') when said blade (18, 12') is connected with said handle (12) said beam producing means (26; 42, 126) including a light source (42) and power supply (26); and

(d) first and second cooperating means (86, 16'; 82) respectively forming parts of the head portion (16) of said handle (12) and the handle connecting segment (20, 18') of said blade (18, 12') for disengagably connecting said disposable blade (18, 12') to said handle (12), characterized in that said second cooperating means (86, 16') include a readily severable, electrically conductive member (86, 16') forming part of the electrical circuit between said power supply (26) and said light source (42, 126), and that said first and second cooperating means (82; 86, 16') include means (82) for severing a substantial part of said electrically conductive member (86) as said blade (18) is disengagably connected to said hanlde (12) for the first time.

2. A laryngoscope (10, 10') according to Claim 1 wherein said electrically conductive member (86, 16') is a relatively thin, elongated strip of metal foil which extends between the head portion (16) of said handle (12) and the handle connecting segment (20, 18') of said blade (18, 12').

3. A laryngoscope (10, 10') according to Claim 2 wherein said first cooperating means (54, 140; 76, 24') includes first and second electrically conductive circuit means (54, 140; 86, 16') forming electrically spaced-apart junctions in said electrical circuit and wherein said strip of foil (86, 16') forms part of said second cooperating means so as to electrically connect said junctions together when said blade (18, 12') is connected to said handle (12).

4. A laryngoscope (10, 10') according to Claim 3 wherein said first circuit means (54, 140) includes a fixed pin (54, 140) serving as one of said junctions and wherein said second cooperating means includes a groove (76, 24') on the side of said foil (86, 16') opposite to the pin (54, 140), said groove (76, 24') cooperating with said pin (54, 140) to carry out said severing of said foil (86, 16') at the time of disengagably connecting said blade (18, 12') to said handle (12).

5. A laryngoscope (10, 10') according to Claim 4 wherein said pin (54, 140) has a circular cross section defined by a given radius and wherein the cross section of said groove (76, 24') is defined by an arc of a circle having approximately the same radius, said arc being sufficiently greater than a half-circle and the groove (76, 24') itself being slightly resilient whereby the groove (76, 24') can interlock around the pin (54, 140), said groove (76, 24') also providing a cutting edge (82) for severing said foil (86, 16'), said cutting edge (82) being shorter than the width of said foil (86, 16').

6. A laryngoscope (10, 10') according to Claim 1 wherein the power supply (26) and the light source (42, 126) are carried by said handle (12) and a light guide (66, 14') is fixedly held by and extending between said handle connecting and tongue holding segments (20, 18'; 22, 20') of said blade (18, 12') so as to be placed in optical alignment with said light source (42, 126) for directing said beam in said predetermined direction when said blade (18, 12') is connected to said handle (12).

7. A laryngoscope (10, 10') according to Claim 6 wherein said blade segments (20, 18'; 22, 20') are integrally formed as a single member having an elongated, open ended passageway (64) configured in an arc of a circle disposed in a single plane, and wherein said light guide (66) is formed from at least one elongated optical fiber and contained within said passageway (64).

8. A laryngoscope (10, 10') according to Claim 7 wherein said integrally formed member is molded plastic.

9. A laryngoscope (10, 10') according to Claim 7 wherein the tongue holding segment (22, 20') of said blade (18, 12') includes a section which extends at an acute angle with said light guide (66, 14') so as to cooperate with said light beam for exposing a patient's throat to view when the laryngoscope (10, 10') is in use and, at the same time, to illuminate the patient's throat with said beam.

10. A laryngoscope (10, 10') according to Claim 7 wherein the end of said light guide (66, 14') adjacent to the handle connecting segment (20, 18') of said blade (18, 12') is flat and extends normal to and coaxial with the axis of said light source (42, 126) when said blade (18, 12') is connected with said handle (12).

11. A laryngoscope (10, 10') according to Claim 7 wherein said handle (12) is substantially straight and wherein said light source (42, 126) is positioned relative to said handle (12) so that its axis is disposed at a 30° angle with a plane normal to said handle (12).

12. A laryngoscope (10, 10') according to Claim 6 including an additional blade (90) having its own tongue holding segment (22) carrying its own light source and a handle connecting segment including means (56, 92) for disengagably connecting said additional blade (90) to said handle (12) in a way which places said last mentioned light source (42) in circuit with said power supply (26) without placing said first mentioned light source in electrical circuit with said power supply (26).

13. A laryngoscope (10, 10') according to Claim 1 wherein the blade connecting head portion (108) of said handle (12) is disengagably connected to the hand gripping portion (14) of said handle (12), said head portion (108) serving as an adapter for handles (12) having original blade connecting head portions which are incompatible with said disposable blade (18, 12').

14. A laryngoscope (10, 10') according to Claim 13 wherein said hand gripping handle portion (14) includes a threaded opening (106A) extending therein from its top end and wherein said head portion includes an elongate hollow at least partially threaded shaft (116) extending into and in threaded engagement with said threaded opening (106A) in said hand gripping portion (14).

15. A laryngoscope (10, 10') according to Claim 14 wherein said beam producing means includes a power supply (26) contained within said hand gripping portion (14) below its threaded opening (106A) and wherein said shaft (116) contains longitudinally electrical contacts (114, 120) at opposite ends thereof and an electrically conductive spring (118) therebetween, the lowermost one (120) of said contacts (114, 120) being in contact with said power supply (26) for placing the other contact (114) in circuit therewith.

16. A laryngoscope (10, 10') according to Claim 15 wherein said head portion (108) includes a main body (108a) having a threaded opening (106A) therethrough for containing said shaft (116) in thread connection therewith.

17. A laryngoscope (10, 10') according to Claim 1 wherein

(a) the blade connecting head portion (16) includes first and second electrically conductive contact members (36, 114; 54, 140) and dielectric means for electrically insulating said members from one another;

(b) connecting said blade (18, 12') to said handle (12) automatically causes said electrically conductive members to engage and electrically connect together said first and second contact members (36, 114; 54 140) thereby automatically energizing said light source (42, 126) without the need for electrical switches.

18. A laryngoscope (10, 10') according to Claim 17 wherein

(a) the laryngoscope blade (18, 12') includes a light guide (66, 14') having a back end located at its handle connecting segment (20, 18') and a front end located at the tongue holding segment (22, 20') said light guide (66, 14') being adapted to receive light at its back end for directing the received light to its front end;

(b) the electrical light source (42, 126) is carried by the blade connecting head portion (16) of said handle (12) and positioned so as to be in optical communication with the back end of said light guide (66, 14') when said blade (18, 12') is connected to said handle (12);

(c) circuit means are provided for electrically connecting said light source (42, 126) and power supply (26) to ground, for electrically connecting said first contact member (36, 114) to the hot side of said power supply (26), and for electrically connecting said second contact member (54, 140) to the hot side of said light source (42, 126), said blade connecting head portion (16) of the handle (12) including a third electrically conductive member including means for electrically connecting said third electrically conductive member to the ground side of said power supply (26) such that when said first and third members are electrically connected across a different light source (42, 126), the latter is automatically energized by said power supply (26), whereby a second type of blade including said second light source and means for engaging said handle in a way which electrically connects said first and third members can be connected with said handle in said last-mentioned way for automatically energizing said second light source when said first-mentioned blade is not connected with said handle.

19. A laryngoscope (10, 10') according to Claim 17 wherein the blade connecting head portion (16) includes a main body (108a) defining an opening (111) therethrough and an elongated stem (108b) including an externally threaded segment (119) configured to extend through the opening (111) in said main body (108a) and into the opening in said hand gripping portion such that the threaded segments (119) of the two cooperate with one another for connecting the stem (108b) to the hand gripping portion (14), said stem (108b) also including shoulder means (116a) engaging said main body (108a) for connecting the latter with said hand gripping portion (14), whereby said blade connecting head portion (16) in its entirety is disengagably connectable with and disengagable from said hand gripping portion (14), said shoulder means (116a) forming part of said stem (108b) including a shoulder (116a) larger than the opening (111) in the main body (108a) of said blade connecting head portion (16) and located at the top end of the stem (108a), said shoulder (116a) including an opening through which a portion of said electrically conductive contact member (114) extends and a slotted top surface (117) for receiving a screwdriver.

20. A laryngoscope (10, 10') according to Claim 1 wherein the handle (12) includes a first light source (42, 126) and the power supply (26); and there is provided, in addition to the first blade carrying a light guide (66, 14') but no light source and having means for mounting the first blade with said handle (12) in a way which causes said power supply (26) to energize the first light source (42, 126) while placing said light guide (66, 14') in optical communication with said last-mentioned light source, a second blade having means including a second light source thereon; and means forming part of said handle (12) and part of said second blade for mounting the second blade with said handle (12) in a way which causes said power supply (26) to energize said second light source without energizing said first light source (42, 126).

21. A laryngoscope (10, 10') according to Claim 1 wherein the handle (12) includes the light source (26), there are provided first electrical contact means fixedly electrically connected with said light source, second electrical contact means fixedly electrically connected with said power supply and means electrically insulating said contacts from one another; and the blade (18, 12') includes means (56, 54; 138, 140) for mounting the blade (18, 12') with said handle (12) in a way which automatically causes said electrical conductive member to engage said first and second contact means in order to electrically connect them together whereby to electrically connect said power supply (26) with said light source (66, 14') for energizing the latter.

22. A laryngoscope (10') according to Claim 1 wherein

(a) the handle connecting end segment (18') includes a straight, open-ended opening (42') extending through a portion thereof and a slot (46'') extending into said opening from one side thereof;

(b) the tongue holding segment (20') is curved and is integrally formed with and extending out from said handle connecting segment (18'), said tongue holding segment (20') including an open-ended, straight opening (40') extending through a part thereof, said opening (40') being entirely closed circumferentially and being oriented in a predetermined way relative to said opening in said tongue holding segment (20'); and

(c) there is provided a flexible, elongated light guide (14') having one end section disposed within the opening (40') in said tongue holding segment (20') and an opposite end segment disposed within the opening (42') in said handle connecting segment (18'), said openings (40', 42') being oriented relative to one another so as to maintain an intermediate section (54') to said light guide (14') in a curved configuration corresponding to an extending along side a section of said curved tongue holding segment (20').

23. A method of making a laryngoscope (10, 10') as indicated in Claim 1, characterized in that for making the blade (18, 12')

(a) a laryngoscope blade body is molded as a single unit having

(i) a handle connecting segment (20, 18'),

(ii) an elongated, curved tongue holding segment (22, 20') connected with and extending out from said handle connecting segment (20, 18'), and

(iii) an open-ended straight opening (40', 42') in each of said segments (20, 18'; 22, 20'), said openings (40', 42') being oriented relative to one another in a predetermined way;

(b) an elongated, flexible light guide (66, 14') is provided and

(c) opposite end segments of said light guide are placed into said openings (40', 42') through said handle connecting segment (20, 18') and tongue holding segment (22, 20'), respectively, said openings (40', 42') being oriented relative to one another such that placement of said end segments in said openings (40', 42') automatically causes an intermediate section (54') of said light guide (66, 14') to be maintained in a curved configuration corresponding to and extending along side a section of said curved tongue holding segment (22, 20').

**Patentansprüche**

1. Laryngoskop (10, 10') mit

(a) einem Griff (12), der einen länglichen Griffabschnitt (14) und ein spatelverbindendes Kopfende (16) aufweist;

(b) einem von dem Griff (12) getrennten Einmalspatel (18, 12'), welcher einen Griffverbindungsabschnitt (20) und einen damit verbundenen länglichen Zungenhalteabschnitt (22, 20'), der sich aus dem Griffverbindungsabschnitt (20, 18') heraus erstreckt, aufweist;

(c) Mitteln (26; 42, 126), die teilweise von dem Griff (12) und teilweise von dem Spatel (18, 12') getragen werden, zum Erzeugen eines Lichtstrahls in einer vorbestimmten Richtung bezüglich des Zungenhalteabschnitts (22, 20') des Spatels (18, 12'), wenn der Spatel (18, 12') mit dem Griff (12) verbunden ist, wobei die Beleuchtungsvorrichtung (26; 42, 126) eine Lichtquelle (42) und eine Energiequelle (26) aufweist; und

(d) einem ersten und einem zweiten mitwirkenden Mittel (86, 16'; 82), die Teile des Kopfendes (16) des Griffes (12) und des Griffverbindungsabschnitts (20, 18') des Spatels (18, 12') bilden, für ein lösbares Verbinden des Einmalspatels (18, 12') mit dem Griff (12),

dadurch gekennzeichnet, daß das zweite mitwirkende Mittel (86, 16') ein leicht trennbares, elektrisch leitendes Teil (86, 16') aufweist, welches einen Bestandteil des elektrischen Stromkreises zwischen der Energiequelle (26) und der Lichtquelle (42, 126) bildet, und das erste und das zweite mitwirkende Mittel (82; 86, 16') ein Mittel (82) für ein Trennen eines wesentlichen Abschnitts des elektrisch leitenden Teils (86) aufweist, wenn der Spatel (18) zum ersten Mal mit dem Griff (12) lösbar verbunden wird.

2. Laryngoskop (10, 10') nach Anspruch 1, worin das elektrisch leitende Teil (86, 16') ein relativ dünner, länglicher Metallfolienstreifen ist, welcher sich zwischen dem Kopfende (16) des Griffs (12) und dem Griffverbindungsabschnitt (20, 18') des Spatels (18, 12') erstreckt.

3. Laryngoskop (10, 10') nach Anspruch 2, worin das erste mitwirkende Mittel (54, 140; 76, 24') erste und zweite elektrisch leitende Stromkreismittel (54, 140; 86, 16') aufweist, welche voneinander beabstandete elektrische Anschlußpunkte in dem Stromkreis bilden, und worin der Folienstreifen (86, 16') einen Teil des zweiten mitwirkenden Mittels bildet, so daß die Anschlußpunkte miteinander elektrisch verbunden sind, wenn der Spatel (18, 12') mit dem Griff (12) verbunden ist.

4. Laryngoskop (10, 10') nach Anspruch 3, worin das erste Stromkreismittel (54, 140) einen fixierten Stift (54, 140) aufweist, der als einer der Anschlußpunkte dient, und worin das zweite mitwirkende Mittel (76, 24') auf der von dem Stift (54, 140) abgewandten Seite der Folie (86, 16') eine Aussparung aufweist, die mit dem Stift (54, 140) zusammenwirkt, um das Trennen der Folie (86, 16') zum Zeitpunkt des lösbaren Verbindens des Spatels (18, 12') mit dem Griff (12) auszuführen.

5. Laryngoskop (10, 10') nach Anspruch 4, worin der Stift (54, 140) einen durch einen gegebenen Radius definierten kreisförmigen Querschnitt hat und der Querschnitt der Aussparung (76, 24') durch einen Kreisbogen mit etwa demselben Radius definiert ist, wobei der Kreisbogen ausreichend größer als ein Halbkreis ist, und die Aussparung (76, 24') selbst leicht elastisch ist, wodurch die Aussparung (76, 24') durch Umgreifen des Stifts (54, 140) ein Verriegeln bewirken kann, wobei die Aussparung (76, 24') auch eine Schneidkante (82) zum Trennen der Folie (86, 16') aufweist, und diese Schneidkante (82) kürzer ist als die Breite der Folie (86, 16').

6. Laryngoskop (10, 10') nach Anspruch 1, worin die Energiequelle (26) und die Lichtquelle (42, 126) von dem Griff (12) getragen werden und ein Lichtleiter (66, 14') durch den Griffverbindungsabschnitt (20, 18') und den Zungenhalteabschnitt (22, 20') des Spatels (18, 12') festgehalten wird und sich zwischen diesen erstreckt, so daß der Lichtleiter in optischer Ausrichtung mit der Lichtquelle (42, 126) angeordnet ist, um den Lichtstrahl in die vorbestimmte Richtung zu lenken, wenn der Spatel (18, 12') mit dem Griff (12) verbunden ist.

7. Laryngoskop (10, 10') nach Anspruch 6, worin die Spatelabschnitte (20, 18'; 22, 20') einstückig als ein Teil mit einem sich längs erstreckenden, offenen Durchgang (64), der in Kreisbogenform in einer einzigen Ebene liegt, ausgebildet sind und der Lichtleiter (66) aus mindestens einer länglichen optischen Faser besteht und innerhalb des Durchgangs (64) angeordnet ist.

8. Laryngoskop (10, 10') nach Anspruch 7, worin das einstückig ausgebildete Teil ein geformter Kunststoff ist.

9. Laryngoskop (10, 10') nach Anspruch 7, worin der Zungenhalteabschnitt (22, 20') des Spatels (18, 12') einen Abschnitt aufweist, der sich in einem spitzen Winkel zu dem Lichtleiter (66, 14') erstreckt, so daß der letztgenannte Abschnitt mit dem Lichtstrahl zusammenwirkt, um den Einblick in die Kehle eines Patienten freizugeben, wenn das Laryngoskop (10, 10') verwendet wird, und um gleichzeitig die Kehle des Patienten mit dem Lichtstrahl zu beleuchten.

10. Laryngoskop (10, 10') nach Anspruch 7, worin das dem Griffverbindungsabschnitt (20, 18') des Spatels (18, 12') benachbarte Ende des Lichtleiters (66, 14') flach ausgebildet ist und sich senkrecht zu und koaxial mit der Achse der Lichtquelle (42, 126) erstreckt, wenn der Spatel (18, 12') mit dem Griff (12) verbunden ist.

11. Laryngoskop (10, 10') nach Anspruch 7, worin der Griff (12) im wesentlichen gerade ausgebildet ist und die Lichtquelle (42, 126) bezüglich des Griffs (12) so angeordnet ist, daß ihre Achse in einem Winkel von 30° zu einer Ebene senkrecht zu dem Griff (12) angeordnet ist.

12. Laryngoskop (10, 10') nach Anspruch 6, welches einen zusätzlichen Spatel (90) mit einem eigenen Zungenhalteabschnitt (22) aufweist, welcher eine eigene Lichtquelle trägt, und mit einem Griffverbindungsabschnitt, welcher Mittel (56, 92) für ein lösbares Verbinden des zusätzlichen Spatels (90) mit dem Griff (12) derart aufweist, daß die letztgenannte Lichtquelle (42) im Stronkreis mit der Energiequelle (26) liegt, ohne die erstgenannte Lichtquelle in einem elektrsichen Stromkreis mit der Energiequelle (26) zu bringen.

13. Laryngoskop (10, 10') nach Anspruch 1, worin das spatelverbindende Kopfende (108) des Griffs (12) lösbar mit dem Griffabschnitt (14) des Griffs (12) verbunden ist, wobei das Kopfende (108) als Anpaßstück für Griffe (12) mit ursprünglichen spatelverbindenden Kopfenden, die mit dem Einmalspatel (18, 12') inkompatibel sind, dient.

14. Laryngoskop (10, 10') nach Anspruch 13, worin der Griffabschnitt (14) eine Gewindeöffnung (106A) aufweist, die sich von seinem oberen Ende aus erstreckt, und worin an dem Kopfende ein länglicher, hohler, mindestens teilweise mit einem Gewinde versehener Schaft (116) vorgesehen ist, der sich in die Gewindeöffnung (106A) in dem Kopfende (14) hinein erstreckt und damit in Schraubverbindung steht.

15. Laryngoskop (10, 10') nach Anspruch 14, worin die Beleuchtungsvorrichtung eine Energiequelle (26) in dem Griffabschnitt (14) unter seiner Gewindeöffnung (106A) aufweist und der Schaft (116) an seinen sich gegenüberliegenden Enden mit längsverlaufenden elektrischen Kontakten (114, 120) und einer elektrisch leitenden Feder (118) dazwischen ausgerüstet ist, wobei der untere Kontakt (120) in Berührung mit der Energiequelle (26) steht, um den anderen Kontakt (114) in einen Stromkreis damit zu bringen.

16. Laryngoskop (10, 10') nach Anspruch 15, worin das Kopfende (108) einen Hauptkörper (108a) mit einer hindurchführenden Gewindeöffnung (106A) aufweist, um den Schaft (116) in Schraubverbindung damit aufzunehmen.

17. Laryngoskop (10, 10') nach Anspruch 1, worin

(a) das spatelverbindende Kopfende (16) einen ersten und einen zweiten elektrisch leitenden Kontakt (36, 114; 54, 140) und ein dielektrisches Mittel zum elektrischen Isolieren der Kontakte voneinander aufweist und

(b) das Verbinden des Spatels (18, 12') mit dem Griff (12) automatisch die elektrisch leitenden Kontakte miteinander in Eingriff bringt und den ersten und den zweiten elektrisch leitenden Kontakt (36, 114; 54, 140) miteinander elektrisch verbindet, und dadurch automatisch die Lichtquelle (42, 126) ohne die Verwendung elektrischer Schalter betrieben wird.

18. Laryngoskop (10, 10') nach Anspruch 17, worin

(a) der Spatel (18, 12') einen Lichtleiter (66, 14') mit einem am Griffverbindungsabschnitt (20, 18') des Spatels (18, 12') gelegenen Hinterende und einem am Zungenhalteabschnitt (22, 20') gelegenen Vorderende aufweist, wobei der Lichtleiter (66, 14') derart ausgebildet ist, daß er an seinem Hinterende Licht aufnimmt und dieses zu seinem Vorderende leitet,

(b) die elektrische Lichtquelle (42, 126) von dem spatelverbindenden Kopfende (16) des Griffs (12) gehalten und so angeordnet ist, daß sie in optischer Verbindung mit dem Hinterende des Lichtleiters (66, 14') steht, wenn der Spatel (18, 12') mit dem Griff (12) verbunden ist, und

(c) Stromkreismittel zum elektrischen Verbinden der Lichtquelle (42, 126) und der Energiequelle (26) mit der Erde, zum elektrischen Verbinden des ersten Kontakts (36, 114) mit der unter Spannung stehenden Seite der Energiequelle (26) und zum elektrischen Verbinden des zweiten Kontakts (54, 140) mit der unter Spannung stehenden Seite der Lichtquelle (42, 126) vorgesehen sind, wobei das spatelverbindende Kopfende (16) des Griffs (12) einen dritten elektrisch leitenden Kontakt aufweist, welcher ein Mittel zum elektrischen Verbinden dieses dritten elektrisch leitenden Kontakts mit der geerdeten Seite der Energiequelle (26) umfaßt, so daß, wenn der erste und der dritte Kontakt über eine andere Lichtquelle (42, 126), elektrisch verbunden sind, diese Lichtquelle automatisch durch die Energiequelle (26) betrieben wird, wobei eine zweite Spatelart mit der zweiten Lichtquelle und dem Mittel für das Einsetzen des Griffs in einer Weise, welche den ersten und den dritten Kontakt elektrisch verbindet, mit dem Griff auf die letztgenannte Weise verbunden werden kann, um die zweite Lichtquelle automatisch zu betreiben, wenn der erstgenannte Spatel mit dem Griff nicht verbunden ist.

19. Laryngoskop (10, 10') nach Anspruch 17, worin das spatelverbindende Kopfende (16) einen Hauptkörper (108a) mit einer durchgehenden Öffnung (111) und einen länglichen Stab (108b) aufweist, der mit einem Abschnitt (119) mit einem Außengewinde versehen ist, welcher sich durch die Öffnung (111) in dem Hauptkörper (108a) und in die Öffnung in dem Griffabschnitt derart erstreckt, daß die Gewindeabschnitte (119) der beiden zusammenwirken, um den Stab (108b) mit dem Griffabschnitt (14) zu verbinden, wobei der Stab (108b) auch eine Schulter (116a) aufweist, die mit dem Hauptkörper (108a) in Eingriff steht, um den letzteren mit dem Griffabschnitt (14) zu verbinden, wobei das spatelverbindende Kopfende (16) als Ganzes mit dem Griffabschnitt (14) lösbar verbindbar ist, die Schulter (116a) einen Teil des Stabes (108b) bildet, der eine Schulter (116a) aufweist, die größer als die Öffnung (111) in dem Hauptkörper (108a) des spatelverbindenden Kopfendes (16) ist und am oberen Ende des Stabes (108a) angeordnet ist, sowie die Schulter (116a) eine Öffnung, durch die sich ein Teil der elektrischen Kontakte (114) hindurch erstreckt, und eine geschlitzte Oberseite (117) zur Aufnahme eines Schraubenziehers aufweist.

20. Laryngoskop (10, 10') nach Anspruch 1, worin der Griff (12) eine erste Lichtquelle (42, 126) und die Energiequelle (26) aufweist und zusätzlich zu dem ersten Spatel, welcher einen Lichtleiter (66, 14'), aber keine Lichtquelle trägt und mit Mitteln ausgerüstet ist zum Zusammensetzen des ersten Spatels mit dem Griff (12) derart, daß die Energiequelle (26) die erste Lichtquelle (42, 126) versorgt, während der Lichtleiter (66, 14') in optischer Verbindung mit der letztgenannten Lichtquelle steht, ein zweiter Spatel vorgesehen ist, der ein Mittel mit einer zweiten Lichtquelle aufweist, sowie Mittel, die einen Teil des Griffs (12) und einen Teil des zweiten Spatels bilden, um den zweiten Spatel mit dem Griff (12) derart zu verbinden, daß die Energiequelle (26) die zweite Lichtquelle versorgt, ohne die erste Lichtquelle (42, 126) zu betreiben.

21. Laryngoskop (10, 10') nach Anspruch 1, worin der Griff (12) die Lichtquelle (26) aufweist, ein erster elektrischer Kontakt, der mit der Lichtquelle fest verbunden ist, ein zweiter elektrischer Kontakt, der mit der Energiequelle elektrisch fest verbunden ist, sowie Mittel zur elektrischen Isolierung der Kontakte voneinander vorgesehen sind, und der Spatel (18, 12') Mittel (56, 54; 138, 140) zum Befestigen des Spatels (18, 12') an dem Griff (12) aufweist, derart, daß der elektrisch leitende Kontakt automatisch mit dem ersten und dem zweiten Kontakt in Eingriff kommt, um sie elektrisch miteinander zu verbinden, wobei die Energiequelle (26) mit der Lichtquelle (66, 14') zum Betreiben der letzteren elektrisch verbunden wird.

22. Laryngoskop (10') nach Anspruch 1, worin

(a) der Griffverbindungsabschnitt (18') eine gerade durchgehende Öffnung (42'), welche sich durch einen Abschnitt derselben hindurch erstreckt, und einen Schlitz (46''), welcher sich von einer Seite der Öffnung in diese hinein erstreckt, aufweist,

(b) der Zungenhalteabschnitt (20') gebogen und mit dem Griffverbindungsabschnitt (18') einstükkig ausgebildet ist und sich aus diesem heraus erstreckt, wobei der Zungenhalteabschnitt (20') eine gerade durchgehende Öffnung (40') aufweist, welche sich über einen Teil desselben erstreckt, wobei die Öffnung (40') in ihrem Umfang völlig geschlossen und in einer vorbestimmten Weise hinsichtlich der Öffnung in dem Zungenhalteabschnitt (20') ausgerichtet ist, und

(c) ein flexibler, länglicher Lichtleiter (14'), dessen eines Ende in der Öffnung (40') des Zungenhalteabschnitts (20') und dessen entgegengesetztes Ende innerhalb der Öffnung (42') des Griffverbindungsabschnitts (18') angeordnet ist, vorgesehen ist, wobei die Öffnungen (40', 42') in bezug zueinander so ausgerichtet sind, daß ein Zwischenabschnitt (54') des Lichtleiters (14') in einer gekrümmten Anordnung festgelegt wird, die einer Seite eines Abschnitts des gekrümmten Zungenhalteabschnitts (20') entspricht und sich entlang derselben erstreckt.

23. Verfahren zum Herstellen eines Laryngoskops (10, 10') nach Anspruch 1, dadurch gekennzeichnet, daß zum Herstellen des Spatels (18, 12')

(a) ein Laryngoskopspatelkörper als eine Einheit mit

(i) einem Griffverbindungsabschnitt (20, 18'),

(ii) einem länglichen, gebogenen Zungenhalteabschnitt (22, 20'), welcher mit dem Griffverbindungsabschnitt (20, 18') verbunden ist und sich aus ihm heraus erstreckt, und

(iii) einer geraden durchgehenden Öffnung (40', 42') in jedem der Abschnitte (20, 18'; 22, 20'), wobei die Öffnungen (40', 42') in vorbestimmter Weise aufeinander ausgerichtet sind, geformt wird,

(b) ein länglicher, flexibler Lichtleiter (66, 14') bereitgestellt wird und

(c) entgegengesetzte Endabschnitte des Lichtleiters durch den Griffverbindungsabschnitt (20, 18') und den Zungenhalteabschnitt (22, 20') in die Öffnungen (40', 42') eingebracht werden, wobei die Öffnungen (40', 42') zueinander so ausgerichtet werden, daß die Positionierung der Endabschnitte in den Öffnungen (40', 42') automatisch einen Zwischenabschnitt (54') des Lichtleiters (66, 14') in einer gekrümmten Anordnung festlegt, die einer Seite eines Abschnitts des gekrümmten Zungenhalteabschnitts (22, 20') entspricht und sich entlang derselben erstreckt.

**Revendications**

1. Un laryngoscope (10, 10') comprenant:

(a) une poignée (12) comportant une partie allongée de saisie manuelle (14) et une partie de tête (16) reliée à une lame;

(b) une lame jetable (18, 12') séparée de ladite poignée (12) et comprenant un segment (20) de liaison avec la poignée et un segment allongé (22, 20') de maintien de langue qui est relié à et fait saillie dudit segment (20, 18') de liaison avec la poignée;

(c) des moyens (26; 42; 126) portés partiellement par ladite poignée (12) et partiellement par ladite lame (18, 12') pour produire un faisceau de laumière dans une direction prédéterminée par rapport au segment de maintien de langue (22, 20') de ladite lame (18, 12') quand ladite lame (18, 12') est reliée à ladite poignée (12), lesdits moyens de production de faisceau (26, 42, 126) comprenant une source de lumière (42) et une source de courant (26); et

(d) des premiers et seconds moyens coopérants (86, 86'; 82) formant respectivement des portions de la partie de tête (16) de ladite poignée (12) et du segment de liaison avec la poignée (20, 18') de ladite lame (18, 12') afin de relier de façon séparable ladite lame jetable (18, 12') avec ladite poignée (12), caractérisé en ce que lesdits seconds moyens coopérants (86, 16') comprennent un élément électriquement conducteur (86, 16') facile à sectionner et faisant partie du circuit électrique existant entre ladite source de courant (26) et ladite source lumineuse (42, 126), et en ce que lesdits premiers et seconds moyens coopérants (82; 86, 16') comprennent un moyen (82) pour sectionner une partie substantielle dudit élément électriquement conducteur (86) lorsque ladite lame (18) est reliée de façon séparable à ladite poignée (12) pour la première fois.

2. Un laryngoscope (10, 10') selon la revendication 1, dans lequel ledit élément électriquement conducteur (86, 16') est une bande allongée et relativement mince d'une feuille métallique qui s'étend entre la partie de tête (16) de ladite poignée (12) et le segment de liaison avec la poignée (20, 20') de ladite lame (18, 12').

3. Un laryngoscope (10, 10') selon la revendication 2, dans lequel lesdits premiers moyens coopérant (54, 140; 76, 24') comprennent des premiers et seconds composants de circuit électriquement conducteurs (54, 140; 86, 16') formant des jonctions électriquement espacées l'une de l'autre dans ledit circuit électrique, et dans lequel ladite bande de feuille métallique (86, 16') fait partie desdits seconds moyens coopérants afinde relier électriquement ensemble lesdites jonctions quand ladite lame (18, 12') est reliée à ladite poignée (12).

4. Un laryngoscope (10, 10') selon la revendication 3, dans lequel ledit premier composant de circuit (54, 140) comprend une broche fixe (54, 140) constituant l'une desdites jonctions, et dans lequel ledit second moyen coopérant comprend une rainure (76, 24') placé sur le côté de ladite feuille (86, 16') qui est opposé à la broche (54, 140), ladite rainure (76, 24') coopérant avec ladite broche (54, 140) pour effectuer ledit sectionnement de ladite feuille (86, 16') au moment de la liaison séparable de ladite lame (18, 12') avec ladite poignée (12).

5. Un laryngoscope (10, 10') selon la revendication 4, dans lequel ladite broche (54, 140) a une section droite circulaire définie par un rayon donné et dans lequel la section droite de ladite rainure (76, 24') est définie par un arc de cercle ayant approximativement le même rayon, ledit arc étant suffisamment plus grand qu'un demi-cercle et la rainure (76, 24') étant légèrement élastique, de telle sorte que cette rainure (76, 24') puisse s'accrocher autour de la broche (54, 140), ladite rainure (76, 24') comportant également une arête de coupe (82) pour sectionner ladite feuille (86, 16'), ladite arête de coupe (82) étant plus courte que la largeur de ladite feuille (86, 16').

6. Un laryngoscope (10, 10') selon la revendication 1, dans lequel la source de courant (26) et la source de lumière (42, 126) sont portées par ladite poignée (12) et un guide de lumière (66, 14') est manitenu en position fixe par et s'étend entre lesdits segments de liaison ave poignée et de maintien de langue (20, 18; 22, 22') de ladite lame (18, 12') de façon à être placé en alignement optique avec ladite source de lumière (42, 126) pour diriger ledit faisceau dans ladite direction prédéterminée quand ladite lame (18, 12') est reliée à ladite poignée (12).

7. Un laryngoscope (10, 10') selon la revendication 6, dans lequel lesdits segments de lame (20, 18'; 22, 20') sont formés unitairement comme un seul élément comportant un passage allongé, ouvert à ses extrémités (64) et profilé en arc de cercle disposé en un seul plan et dans lequel ledit guide de lumière (66) est formé à partir d'au moins une fibre optique allongée et disposée à l'intérieur dudit passage (64).

8. Un laryngoscope (10, 10') selon la revendication 7, caractérisé en ce que ledit élément formé unitairement est moulé en matière plastique.

9. Un laryngoscope (10, 10') selon la revendication 7, dans lequel le segment de maintien de langue (22, 20') de ladite lame (18, 12') comprend une section qui est orientée selon un angle aigu par rapport audit guide de lumière (66, 14') afin de coopérer avec ledit faisceau lumineux pour exposer la gorge d'un patient à la vue quand le laryngoscope (10, 10') est en service et, en même temps, pour éclairer la gorge du patient avec ledit faisceau.

10. Un laryngoscope (10, 10') selon la revendication 7, dans lequel l'extrémité dudit guide de lumière (66, 14') qui est adjacente au segment de liaison avec la poignée (20, 18') de ladite lame (18, 12') est plane et s'étend normalement et coaxialement à l'axe de ladite source de lumière (42, 126) quand ladite lame (18, 12') est reliée à ladite poignée (12).

11. Un laryngoscope (10, 10') selon la revendication 7, dans lequel ladite poignée (12) est sensiblement rectiligne et dans lequel ladite source lumineuse (42, 126) est positionnée par rapporte à ladite poignée (12) de telle sorte que son axe soit orienté d'un angle de 30° par rapport à un plan perpendiculaire à ladite poignée (12).

12. Un laryngoscope (10, 10') selon la revendication 6, comprenant une lame additionnelle (90) pourvue de son propre segment de maintien de langue (22) portant sa propre source de lumière et d'un segment de liaison avec la poignée comprenant des moyens (56, 92) pour relier de façon séparable ladite lame additionnelle (90) avec ladite poignée (12) d'une manière qui place la source lumineuse (42) mentionnée en dernier en circuit avec ladite source de courant (26) sans placer ladite source lumineuse mentionnée en premier en liaison électrique avec ladite source de courant (26).

13. Un laryngoscope (10, 10') selon la revendication 1, dans lequel la partie de tête (108), assurant la liaison avec la lame, de ladite poignée (12) est reliée de façon séparable à ladite partie de saisie manuelle (14) de ladite poignée (12), ladite partie de tête (108) servant d'adaptateur pour des poignées (12) comportant des parties de tête d'origine destinées à assurer la liaison avec la lame et qui sont incompatibles avec ladite lame jetable (18, 12').

14. Un laryngoscope (10, 10') selon la revendication 13, dans lequel ladite partie de saisie manuelle (14) de la poignée comporte un trou fileté (106A) s'étendant à partir de son extrémité supérieure, et dans lequel ladite partie de tête comporte une tige creuse allongée et au moins partiellement filetée (116) qui pénètre et est vissée dans ledit trou (106A) ménagé dans ladite partie de saisie manuelle (14).

15. Un laryngoscope (10, 10') selon la revendication (14), caractérisé en ce que ledit moyen de production de faisceau comprend une source de courant (26) logée à l'intérieur de ladite partie de saisie manuelle (14) en dessous de son trou fileté (106A) et dans lequel ladite tige (116) contient des contacts électriques (114, 120) disposés longitudinalement à ses extrémités opposées et un ressort électriquement conducteur (118) disposé entre eux, celui (120) desdits contacts (114, 120) qui est placé en bas étant en contact avec ladite source de courant (26) pour assurer la liaison électrique avec l'autre contact (114).

16. Un laryngoscope (10, 10') selon la revendication 15, dans lequel ladite partie de tête (108) comprend un corps principal (108a) pourvu d'un trou fileté (106A) le traversant de manière à maintenir la tige (16) en liaison vissée avec elle.

17. Un laryngoscope (10, 10') selon la revendication 1, dans lequel:

(a) la partie de tête (16) assurant la liaison avec la lame comprend un premier et un second élément de contact électriquement conducteur (36, 114; 54, 140) et un moyen diélectrique pour isoler électriquement lesdits éléments de contact l'un de l'autre.

(b) une liaison de ladite lame (18, 12') avec ladite poignée (12) fait automatiquement en sorte que lesdits éléments électriquement conducteurs s'appliquent contre et relient électriquement ensemble lesdits premier et second éléments de contact (36, 14; 54, 140) en produisant ainsi automatiquement une excitation de ladite source lumineuse (42, 126) sans l'obligation de prévoir des interrupteurs électriques.

18. Un laryngoscope (10, 10') selon la revendication 17, dans lequel:

(a) la lame (18, 12') du laryngoscope comprend un guide de lumière (66, 14') comportant une extrémité arrière placée sur son segment (20, 18') de liaison avec la poignée et une extrémité avant placée sur le segment (22, 20') de maintien de langue, ledit guide de lumière (66, 14') étant adapté pour recevoir de la lumière à son extrémité arrière afin de diriger la lumière reçue vers son extrémité avant;

(b) la source lumineuse électrique (42, 126) est supportée par la partie de tête (16), assurant la liaison avec la lame, de ladite poignée (12) et elle est positionnée de façon à être en communication optique avec l'extrémité arrière dudit guide de lumière (66, 14') quand ladite lame (18, 12') est reliée à ladite poignée (12);

(c) il est prévu des composants de circuit pour relier électriquement ladite source de lumière (42, 126) et ladite source de courant (26) à la masse, pour relier électriquement ledit premier élément (36, 114) avec le côté d'excitation de ladite source de courant (26) et pour relier électriquement ledit second élément de contact (54, 140) avec le côté d'excitation de ladite source lumineuse (42, 126), ladite partie de tête (16), assurant la liaison avec la lame, de la poignée (12) comprenant un troisième élément électriquement counducteur qui est pourvu de moyens servant à relier électriquement ledit troisième élément électriquement conducteur avec le côté-masse de ladite source de courant 26 de telle sorte que, quand lesdits premier et troisième éléments sont reliés électriquement aux bornes d'une source lumineuse différente (42, 126), cette dernière soit automatiquement excitée par ladite source de courant (26), afin qu'un second type de lame comprenant ladite seconde source lumineuse et un moyen d'engagement de ladite poignée d'une manière qui assure la liaison électrique desdits premier et troisième éléments, puissent être reliés avec ladite poignée de la manière mentionnée en dernier en vue d'une excitation automatique de ladite seconde source de lumière quand ladite lame mentionnée en premier n'est pas reliée à ladite poignée.

19. Un laryngoscope (10, 10') selon la revendication 17, dans lequel la partie de tête (16), assurant la liaison avec la lame, comprend un corps principal (108a) définissant un trou traversant (111) et une tige allongée (108b) comportant un segment fileté extérieurement (119), qui est agencé pour s'engager dans ledit trou (111) dudit corps principal (108a) et dans le trou ménagé dans ladite partie de saisie manuelle, de telle sorte que les segments filetés (119) des deux parties coopèrent l'un avec l'autre pour relier la tige (108b) à la partie de saisie manuelle (14), ladite tige (108b) comportant également un épaulement (116a) s'appliquant contre ledit corps principal (108a) pour relier ce dernier à ladite partie de saisie manuelle (14), de telle sorte que ladite partie de tête (16) dans son ensemble puisse être reliée de façon démontable à la partie de saisie manuelle (14), ladite partie à épaulement (116a) qui fait partie de ladite tige (108b) étant pourvue d'un épaulement (116a) plus grand que le trou (111) ménagé dans le corps principal (108a) de ladite partie de tête (16) assurant la liaison avec la lame et étant placée à l'extrémité supérieure de ladite tige (108a), ledit épaulement (116a) comportant une ouverture au travers de laquelle peut passer une partie dudit élément de contact électriquement conducteur (114) ainsi qu'une surface supérieure encochée (117) pour la réception d'un tournevis.

20. Un laryngoscope (10, 10') selon la revendication 1, dans lequel la poignée (12) comprend une première source de lumière (42, 126) et la source de courant (26); et il est prévu, en addition à la première lame portant un guide de lumière (66, 14') mais pas de source de lumière et comportant un moyen pour le montage de la première lame avec ladite poignée (12) d'une manière faisant en sorte que ladite source de courant (26) excite la première source de lumière (42, 126) tout en plaçant ledit guide de lumière (66, 14') en communication optique avec ladite source de lumière mentionnée en dernier, une seconde lame comportant un moyen pourvu d'une seconde source de lumière; ainsi qu'un moyen faisant partie de ladite poignée (12) et faisant partie de ladite seconde lame pour le montage de la seconde lame avec la poignée (12) d'une manière faisant en sorte que ladite source de courant (26) excite ladite seconde source de lumière sans exciter ladite première source de lumière (42, 126).

21. Un laryngoscope (10, 10') selon la revendication 1, dans lequel la poignée (12) comprend la source de lumière (26), il est prévu un premier contact électrique relié électriquement de façon fixe à ladite source de lumière, un second contact électrique relié électriquement de façon fixe à ladite source de courant et un moyen isolant électriquement lesdits contacts l'un de l'autre; et la lame (18, 12') comprend des moyens (56, 54; 138, 140) pour le montage de la lame (18, 12') avec ladite poignée (12) d'une manière qui fasse automatiquement en sorte que ledit élément électriquement conducteur s'applique contre lesdits premier et second contacts pour les relier électriquement ensemble en vue de raccorder électriquement ladite source de courant (26) à ladite source de lumière (66, 14') afin d'assurer son excitation.

22. Un laryngoscope (10') selon la revendication 1, dans lequel:

(a) le segment extrême (18') assurant la liaison avec la poignée comprend un trou rectiligne (42'), ouvert à ses extrémités et s'étendant au travers d'une partie du segment, ainsi qu'une fente (46'') s'étendant jusque dans ledit trou à partir d'un côté du segment;

(b) le segment de maintien de langue (20') est incurvé et est formé unitairement avec le segment (18') de liaison avec la poignée en faisant saillie extérieurement de celui-ci, ledit segment de maintien de langue (20') comprenant un trou rectiligne (40') ouvert à ses extrémités et traversant une partie dudit segment, ledit trou (40') étant entièrement fermé circonférentiellement et étant orienté d'une manière prédéterminée par rapport audit trou ménagé dans ledit segment de maintien de langue (20'); et

(c) il est prévu un guide de lumière (14') flexible, allongé, et comportant une section extrême qui est disposée à l'intérieur du trou (40') ménagé dans ledit segment de maintien de langue (20') ainsi qu'un segment extrême opposé, disposé à l'intérieur du trou (42') ménagé dans ledit segment (18') de liaison avec la poignée, lesdits trous (40', 42') étant orientés l'un par rapport à l'autre de façon à maintenir une section intermédiaire (54') dudit guide de lumière (14') dans une configuration incurvée en correspondance à, et le long d'un côté d'une section dudit segment incurvé de maintien de langue (20').

23. Un procédé de fabrication d'un laryngoscope (10, 10') tel que revendiqué dans la revendication 1, caractérisé en ce que, pour la fabrication de la lame (18, 12'):

(a) un corps de lame de laryngoscope est moulé sous la forme d'une seule pièce comprenant:

(i) un segment (20, 18') de liaison avec la poignée,

(ii) un segment incurvé et allongé (22, 20') de maintien de langue qui est relié au segment (20, 18') de liaison avec la poignée et s'étend à l'extérieur de celui-ci, et

(iii) un trou rectiligne (40', 42'), ouvert à ses extrémités et ménagé dans chacun desdits segments (20, 18'; 22, 20'), lesdits trous (40', 42') étant orientés l'un par rapport à l'autre d'une manière prédéterminée;

(b) il est prévu un guide de lumière allongé et flexible (66, 14'), et

(c) des segments extrêmes opposés dudit guide de lumière sont engagés dans lesdits trous (40', 42') ménagés respectivement dans ledit segment de liaison avec la poignée (20, 18') et ledit segment de maintien de langue (22, 20'), lesdits trous (40', 42') étant orientés l'un par rapport à l'autre de telle sorte qu'une mise en place desdits segments extrêmes dans lesdits trous (40', 42') fasse automatiquement en sorte qu'une section intermédiaire (54') dudit guide de lumière (66, 14') soit maintenue dans une configuration incurvée correspondant à et s'étendant le long d'un côté d'une section dudit segment incurvé de maintien de langue (22, 20').

FIG. I

FIG. 4

FIG. 3

FIG. 2

FIG. 6

FIG. 5

FIG. 7

FIG. 8

FIG. 9

FIG. 10

FIG. 11A

FIG. 11

FIG. 12

FIG. 13

FIG. 14

FIG. 15

FIG. 15A

FIG. 15B

FIG. 16

FIG. 18

FIG. 19

FIG. 20

FIG. 17

FIG. 22

FIG. 23

FIG. 21